# EUROPEAN PATENT APPLICATION

(11) **EP 4 603 114 A1**
(43) Date of publication of application: **20.08.2025**
(21) Application number: 23891077.2
(22) Date of filing: 19.06.2023
(51) Int. Cl.: A61M 5/00

(54) **GASKET HOLDER, GASKET HOLDER SET AND GASKET HOUSING BODY**

(30) Priority: 14.11.2022 JP 2022181791
(71) Applicant: Terumo Kabushiki Kaisha, Tokyo 151-0072 (JP)
(72) Inventor: SAKAGUCHI, Yusuke, Fujinomiya-shi, Shizuoka 418-0004 (JP); GOTO, Kazunori, Fujinomiya-shi, Shizuoka 418-0004 (JP); SHINJO, Masahiko, Fujinomiya-shi, Shizuoka 418-0004 (JP); KAKU, Yusuke, Fujinomiya-shi, Shizuoka 418-0004 (JP)
(74) Representative: Prüfer & Partner mbB Patentanwälte · Rechtsanwälte
(86) International application number: PCT/JP2023/022589
(87) International publication number: WO 2024/105915

(57) **Abstract**

The present invention relates to a gasket holding tool (38), a gasket holding tool set (30), and a gasket housing body (10). A holding structure (42) of a gasket holding tool (38) includes an insertion hole (51), a first holding portion (52) that abuts on a proximal-end-side outer peripheral surface (27) of a gasket (12), and a second holding portion (54) that abuts on a surface (25b) of a protruding portion (18) of the gasket (12) facing in a distal direction. The first contact portion (53) of the first holding portion (52) and the second contact portion (62) of the second holding portion (54) are located so as to be offset from each other in the thickness direction of the base plate (40). The holding structure (42) is not in contact with the distal-end-side protruding portion (16) in the holding state of the gasket (12).

## Description

### Technical Field

The present invention relates to a gasket holding tool, a gasket holding tool set, and a gasket housing body.

### Background Art

For example, WO 2015/012206 discloses a gasket to be inserted into an outer tube of a syringe. The gasket includes a gasket body and an annular distal-end-side protruding portion that protrudes radially outward from an outer peripheral surface of the gasket body and is in liquid-tight contact with an inner peripheral surface of the outer tube.

### Summary of Invention

Incidentally, for example, in a case where the gasket as described above is inserted into the cylindrical portion of the gasket holding tool and held before the gasket is plugged into the outer tube, there is a possibility that the distal-end-side protruding portion as the sealing portion comes into contact with the inner peripheral surface of the cylindrical portion and is damaged.

An object of the present invention is to solve the problem described above.
(1) A first aspect of the present disclosure is a gasket holding tool for holding a gasket to be inserted into an outer tube of a syringe, the gasket including: a gasket body; an annular sealing portion provided on an outer peripheral surface of the gasket body and in liquid-tight or gas-tight contact with an inner peripheral surface of the outer tube; and an annular protruding portion protruding radially outward from a portion of the outer peripheral surface of the gasket body in a proximal direction with respect to the sealing portion, the gasket holding tool including: a base plate; and a holding structure that is provided on the base plate and holds the gasket, in which the holding structure includes: an insertion hole through which the gasket is inserted; a first holding portion including a first contact portion that abuts on a proximal-end-side outer peripheral surface in the proximal direction with respect to the protruding portion of the outer peripheral surface of the gasket body in a state where the gasket is inserted into the insertion hole; and a second holding portion including a second contact portion that abuts on a surface of the protruding portion facing a distal direction in a state where the gasket is inserted into the insertion hole, the first contact portion and the second contact portion are located so as to be offset from each other in a thickness direction of the base plate, and the holding structure is not in contact with the sealing portion in a holding state of holding the gasket.
   According to such a configuration, by inserting the gasket into the insertion hole from the proximal direction of the gasket body, the gasket can be held by the first holding portion and the second holding portion without the sealing portion coming into contact with the holding structure. In addition, the holding structure is not in contact with the sealing portion in the holding state of the gasket. As a result, the gasket can be held by the gasket holding tool while suppressing damage of the sealing portion due to contact with the holding structure. Further, since the first contact portion abuts on the proximal-end-side outer peripheral surface of the gasket body and the second contact portion abuts on the surface of the protruding portion of the gasket in the distal direction, the gasket can be stably held.
(2) In the gasket holding tool according to item (1), a plurality of the first holding portions and a plurality of the second holding portions may be provided at intervals along a circumferential direction of the insertion hole, a first gap may be formed between the first holding portions adjacent to each other in the holding state, a second gap may be formed between the second holding portions adjacent to each other in the holding state, and steam or gas may flow through the first gap and the second gap when the gasket is subjected to high-pressure steam sterilization or gas sterilization.
   According to such a configuration, the gasket held by the gasket holding tool can be efficiently sterilized with high-pressure steam or gas. In addition, the gasket can be more stably held by the plurality of first holding portions and the plurality of second holding portions.
(3) In the gasket holding tool according to item (2), a plurality of the first holding portions and a plurality of the second holding portions may be alternately arranged so as not to overlap each other when viewed from the thickness direction of the base plate.
   According to such a configuration, the steam can be smoothly circulated around the gasket.
(4) In the gasket holding tool according to any one of items (1) to (3), one of the first holding portion and the second holding portion may be provided on an inner surface of the insertion hole, and the other one of the first holding portion and the second holding portion may extend from the base plate in the thickness direction of the base plate.
   According to such a configuration, the holding structure can be formed compactly.
(5) In the gasket holding tool according to any one of items (1) to (4), the second contact portion may have a tapered surface inclined in a direction away from a center line of the insertion hole toward a direction opposite to the first contact portion.
   According to such a configuration, by bringing the protruding portion of the gasket into contact with the tapered surface, the protruding portion easily gets over the second contact portion.
(6) The gasket holding tool according to any one of items (1) to (5) may further include a leg portion protruding downward from the base plate, in which a lower end of the leg portion is located below a lower end of the gasket in the holding state.
   According to such a configuration, in a case where the plurality of gasket holding tools are disposed in an overlapping manner, it is possible to prevent the adjacent gasket holding tools from coming into contact with the gasket.
(7) In the gasket holding tool according to item (6), a leg insertion recess into which a lower end of the leg portion is insertable may be provided at an upper end of the leg portion.
   According to such a configuration, in a case where the plurality of gasket holding tools are disposed in an overlapping manner, the lower end portion of the leg portion can be easily located by being inserted into the leg insertion recess.
(8) In the gasket holding tool according to item (6) or (7), the leg portion may be formed in a tubular shape, and a notch for discharging water inside the leg portion may be formed at a lower end of the leg portion.
   According to such a configuration, in a case where the gasket is sterilized with high-pressure steam, the water (liquid water) in the leg portion can be discharged to the outside from the notch.
(9) In the gasket holding tool according to any one of items (1) to (8), an outer peripheral rib protruding in the thickness direction of the base plate may be provided on an outer peripheral portion of the base plate.
   According to such a configuration, the rigidity of the outer peripheral portion of the base plate can be improved by the outer peripheral rib.
(10) In the gasket holding tool according to item (9), a through-hole for transportation may be formed in a portion of the base plate adjacent to the outer peripheral rib.
   According to such a configuration, it is possible to easily transport the gasket holding tool by inserting a human finger, a claw of a robot arm, or the like into the through-hole. Further, the periphery of the portion of the base plate where the through-hole is formed can be reinforced by the outer peripheral rib.
(11) A second aspect of the present disclosure is a gasket holding tool set including: a gasket holding tool according to any one of items (1) to (10); and the gasket.
(12) A third aspect of the present disclosure is a gasket housing body including: a gasket holding tool set according to item (11); a container that has an opening opened upward and houses the gasket holding tool set; and a sealing member that seals the opening of the container, in which the sealing member is formed such that steam or gas for performing high-pressure steam sterilization or gas sterilization of the gasket can pass therethrough.
   According to such a configuration, the gasket held by the gasket holding tool can be sterilized with high-pressure steam or gas.
(13) In the gasket housing body according to item (12), the container may include: a bottom wall having a bottom surface forming a housing chamber of the container; a peripheral wall provided on an outer peripheral portion of the bottom wall; and a bottom surface rib protruding upward from the bottom surface, and the bottom surface rib may be provided so as to partition the bottom surface into a plurality of regions.
   According to such a configuration, the rigidity of the bottom wall can be improved by the bottom surface rib. Therefore, thermal deformation of the bottom wall (warpage of the bottom wall) during high-pressure steam sterilization can be suppressed. In addition, water (liquid water) generated at the time of high-pressure steam sterilization of the gasket can be dispersed and stored in the plurality of regions. That is, it is possible to suppress biased accumulation of water (liquid water) in the bottom surface.
(14) In the gasket housing body according to item (13), the bottom surface rib may be formed such that each of the plurality of regions has a polygonal shape in top view.
   According to such a configuration, the rigidity of the bottom wall can be effectively improved by the bottom surface rib.
(15) In the gasket housing body according to item (14), the bottom surface rib may be formed such that each of the plurality of regions has a hexagonal shape in top view.
   According to such a configuration, the rigidity of the bottom wall can be more effectively improved by the bottom surface rib. In addition, the plurality of regions can be arranged on the bottom surface in a well-balanced manner.
(16) In the gasket housing body according to item (15), the bottom surface rib may be formed such that one of the plurality of regions is located at a center of the bottom wall, and a center of the bottom wall may be located in the one region.
   According to such a configuration, since the bottom surface rib can be arranged on the bottom surface in a more balanced manner, it is possible to suppress the variation in the magnitude of the rigidity of the bottom wall. This makes it possible to further suppress thermal deformation of the bottom wall (warpage of the bottom wall) at the time of high-pressure steam sterilization.
(17) In the gasket housing body according to item (13), the bottom wall may be located at an intermediate portion of the peripheral wall in a vertical direction.
   According to such a configuration, the rigidity of the peripheral wall can be effectively improved by the bottom wall as compared with the case where the bottom wall is located at the lower end of the peripheral wall.
(18) In the gasket housing body according to item (13), the container may include: a flange portion extending outward from an upper end portion of the peripheral wall; and a flange rib protruding in a vertical direction from the flange portion.
   According to such a configuration, the rigidity of the upper end portion of the peripheral wall can be improved by the flange portion and the flange rib.
(19) A fourth aspect of the present disclosure is a gasket housing body including: a gasket holding tool set including a gasket holding tool according to item (6) and the gasket; a container that has an opening opened upward and houses the gasket holding tool set; and a sealing member that seals the opening of the container, in which the sealing member is formed such that steam or gas for performing high-pressure steam sterilization or gas sterilization of the gasket can pass therethrough, the container includes: a bottom wall having a bottom surface forming a housing chamber of the container; a peripheral wall provided on an outer peripheral portion of the bottom wall; and a support portion protruding upward from the bottom wall, and a support recess into which a lower end portion of the leg portion is insertable is provided at an upper end portion of the support portion.
   According to such a configuration, the weight of the gasket holding tool set can be supported by the support portion protruding from the bottom wall. By inserting the lower end portion of the leg portion of the gasket holding tool into the support recess, the gasket holding tool can be easily located with respect to the container.
(20) In the gasket housing body according to item (19), the leg portion may be formed in a tubular shape, a notch for discharging water inside the leg portion may be formed at a lower end portion of the leg portion, a lower end surface of the leg portion may abut on a bottom surface of the support recess, and a drain hole for guiding the water discharged from the notch to the outside of the support portion may be formed in a side wall forming the support recess.
   According to such a configuration, the water (liquid water) in the leg portion can be discharged through the notch and the drain hole at the time of high-pressure steam sterilization of the gasket.
(21) In the gasket housing body according to item (20), the container may include a container leg portion that extends downward from the support portion below the bottom wall and receives a load acting on the support portion.

According to such a configuration, since the load acting on the support portion can be received by the container leg portion, it is possible to suppress the support portion and the bottom wall from being deformed by the weight of the gasket holding tool set.

According to the present invention, the gasket can be held by the gasket holding tool while suppressing damage of the sealing portion due to contact with the holding structure.

### Brief Description of Drawings

Fig. 1 is a perspective view of a gasket housing body according to an embodiment of the present invention.
Fig. 2 is an exploded perspective view of the gasket housing body of Fig. 1.
Fig. 3 is a longitudinal cross-sectional view taken along line III-III of the gasket housing body of Fig. 1.
Fig. 4 is a plan view of the holding structure of Fig. 3.
Fig. 5 is a longitudinal cross-sectional view taken along line V-V in Fig. 4.
Fig. 6 is an enlarged view of the holding structure and the gasket of Fig. 3.
Fig. 7 is a longitudinal cross-sectional view taken along line VII-VII of Fig. 6.
Fig. 8 is a plan view of a gasket and a holding mechanism.
Fig. 9 is a transverse cross-sectional view taken along line IX-IX in Fig. 6.
Fig. 10 is an enlarged view of the leg portion and the support portion of Fig. 3.
Fig. 11 is a plan view of the container of Fig. 2.
Fig. 12 is a longitudinal cross-sectional view of a syringe including the gasket of Fig. 3.
Fig. 13 is a cross-sectional view illustrating a holding structure according to a first modification.
Fig. 14 is a cross-sectional view illustrating a holding structure according to a second modification.

### Description of Embodiments

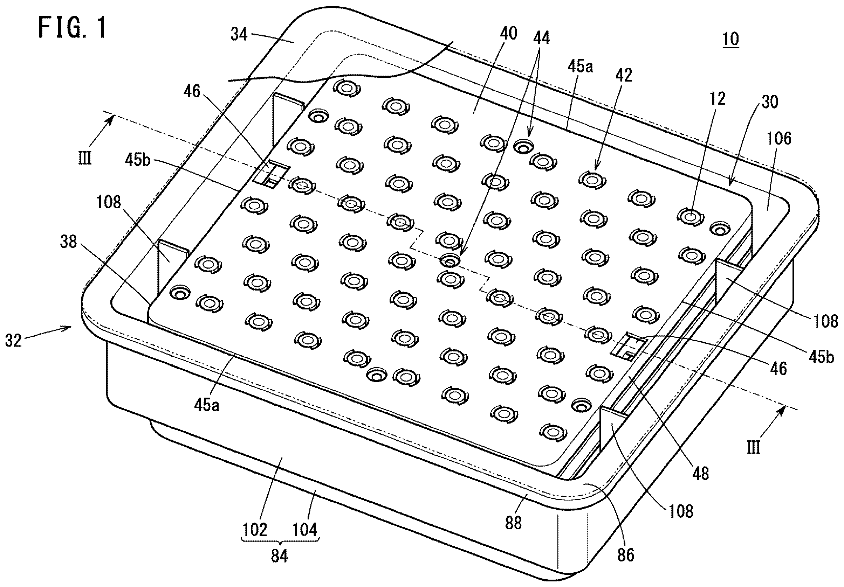

As illustrated in Figs. 1 to 3, a gasket housing body 10 according to an embodiment of the present invention is a medical device for simultaneously performing high-pressure steam sterilization (autoclave treatment) on a large number of gaskets 12. First, a syringe 300, which is a final product including the gasket 12, will be described.

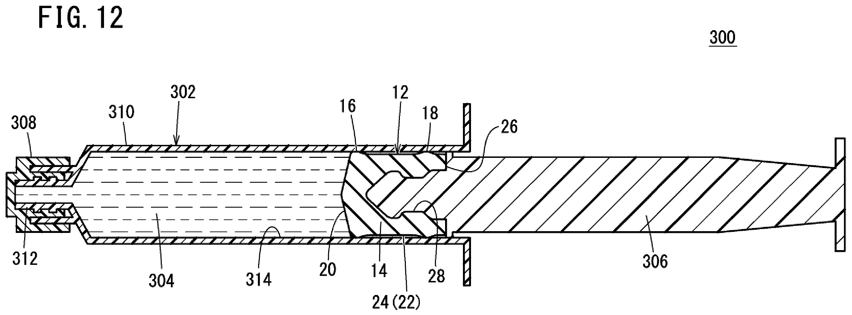

As illustrated in Fig. 12, the syringe 300 is configured as, for example, a prefilled syringe. However, as long as the syringe 300 includes the gasket 12, the structure and use thereof are not limited. That is, the syringe 300 may be a blood sampling syringe or the like.

The syringe 300 includes a syringe body 302, a drug 304, a gasket 12, a plunger 306, and a cap 308. The syringe body 302 includes a cylindrical outer tube 310 (barrel) and a nozzle 312 forming a distal end portion of the outer tube 310. The gasket 12 is disposed inside the outer tube 310 so as to be slidable in the axial direction of the syringe body 302. A syringe chamber 314 filled with the drug 304 is formed in the distal direction of the gasket 12 in the outer tube 310.

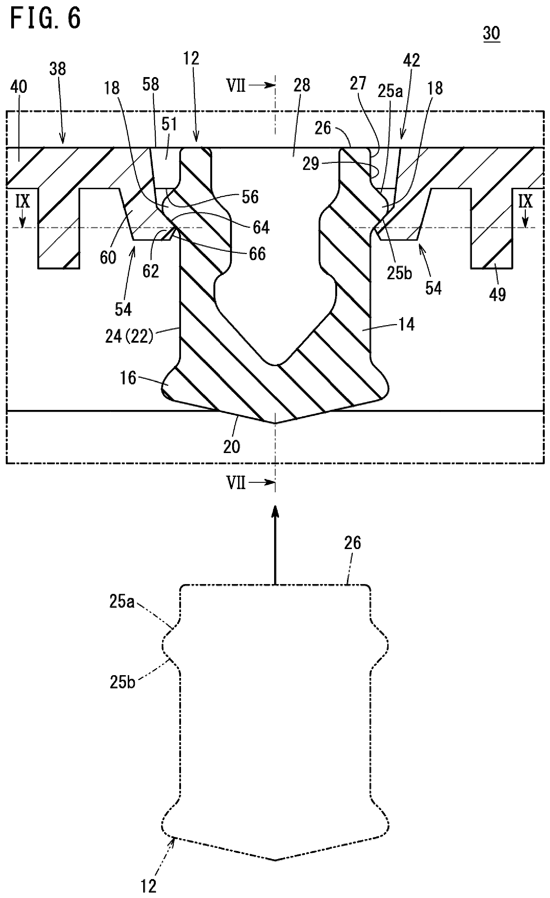

In Figs. 6 and 12, the gasket 12 includes a gasket body 14, a distal-end-side protruding portion 16 (sealing portion), and a proximal-end-side protruding portion 18 (protruding portion). The gasket body 14 extends along the axial direction of the syringe body 302. A distal end surface 20 of the gasket body 14 seals the syringe chamber 314 from the proximal direction (see Fig. 12). The distal end surface 20 of the gasket body 14 is tapered in diameter in the distal direction.

Each of the distal-end-side protruding portion 16 and the proximal-end-side protruding portion 18 protrudes radially outward from an outer peripheral surface 22 of the gasket body 14 and annularly extends. The distal-end-side protruding portion 16 is provided at a distal end portion of the outer peripheral surface 22 of the gasket body 14. In other words, the distal-end-side protruding portion 16 is located in the distal direction from the center in the axial direction of the gasket body 14 and is not located in the proximal direction from the center in the axial direction of the gasket body 14. The distal-end-side protruding portion 16 is located in a range of 1/4 of the entire length in the axial direction of the gasket body 14 in the proximal direction from the distal end of the gasket body 14.

The distal-end-side protruding portion 16 is formed to be narrowed radially outward (so that the width in the axial direction of the gasket body 14 is narrowed). The protruding end surface of the distal-end-side protruding portion 16 is formed in an arc shape when viewed from a direction orthogonal to the axial direction of the gasket body 14 (see Fig. 6). The outer surface of the distal-end-side protruding portion 16 is connected to the distal end surface 20 of the gasket body 14 without a step.

As illustrated in Fig. 12, the distal-end-side protruding portion 16 is in liquid-tight or gas-tight contact with the inner surface of the outer tube 310. That is, the syringe chamber 314 is kept liquid-tight or gas-tight by the distal-end-side protruding portion 16 of the gasket 12. A lubricant is applied to an outer surface of the gasket 12 including the distal-end-side protruding portion 16. Accordingly, the gasket 12 can smoothly slide on the inner surface of the outer tube 310 in the axial direction of the gasket body 14.

In Figs. 6 and 12, the proximal-end-side protruding portion 18 is located in the proximal direction from the center in the axial direction of the gasket body 14 and is not located in the distal direction from the center in the axial direction of the gasket body 14. The proximal-end-side protruding portion 18 is located in the distal direction with respect to the proximal end of the gasket body 14. The proximal-end-side protruding portion 18 is formed to be narrowed radially outward (so that the width in the axial direction of the gasket body 14 is narrowed).

The protruding end surface of the proximal-end-side protruding portion 18 is formed in an arc shape when viewed from a direction orthogonal to the axial direction of the gasket body 14 (see Fig. 6). In Fig. 12, the proximal-end-side protruding portion 18 is in contact with the inner surface of the outer tube 310. The proximal-end-side protruding portion 18 suppresses inclination of the axis of the gasket body 14 with respect to the axis of the syringe body 302.

As illustrated in Figs. 6 and 12, an intermediate outer peripheral surface 24 between the distal-end-side protruding portion 16 and the proximal-end-side protruding portion 18 in the outer peripheral surface 22 of the gasket body 14 is located radially inward of the distal-end-side protruding portion 16 and the proximal-end-side protruding portion 18. That is, the gasket 12 has a shape in which the intermediate portion in the axial direction of the gasket body 14 is recessed radially inward. The length of the intermediate outer peripheral surface 24 along the axial direction of the gasket body 14 (the interval between the distal-end-side protruding portion 16 and the proximal-end-side protruding portion 18) is 1/3 or more of the entire length of the gasket body 14 in the axial direction.

In Fig. 12, the plunger 306 presses the gasket 12 in the distal direction. A distal end portion of the plunger 306 is connected to a connection hole 28 formed in the proximal end surface 26 of the gasket 12. The cap 308 seals the distal end opening of the nozzle 312.

In Figs. 1 to 3, the gasket housing body 10 includes a plurality of gasket holding tool sets 30, a container 32, and a sealing member 34.

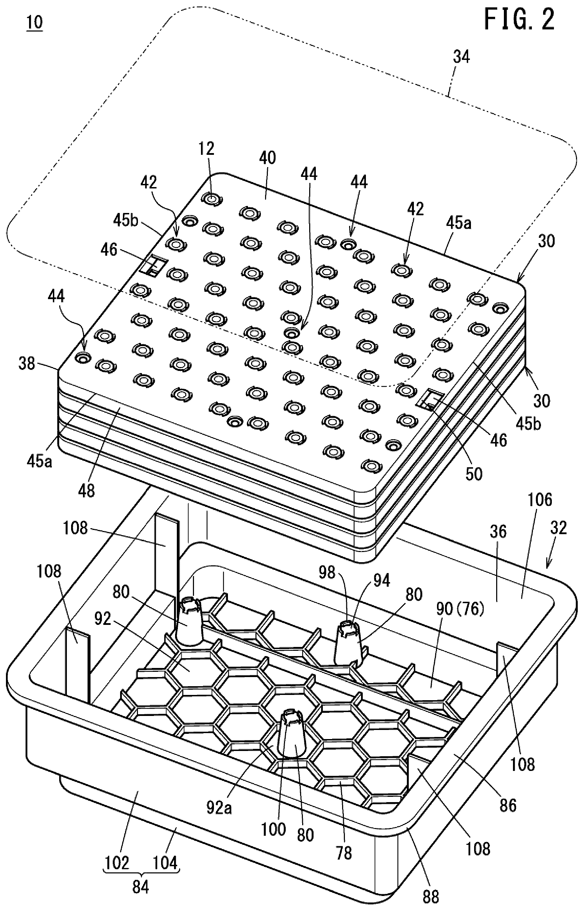

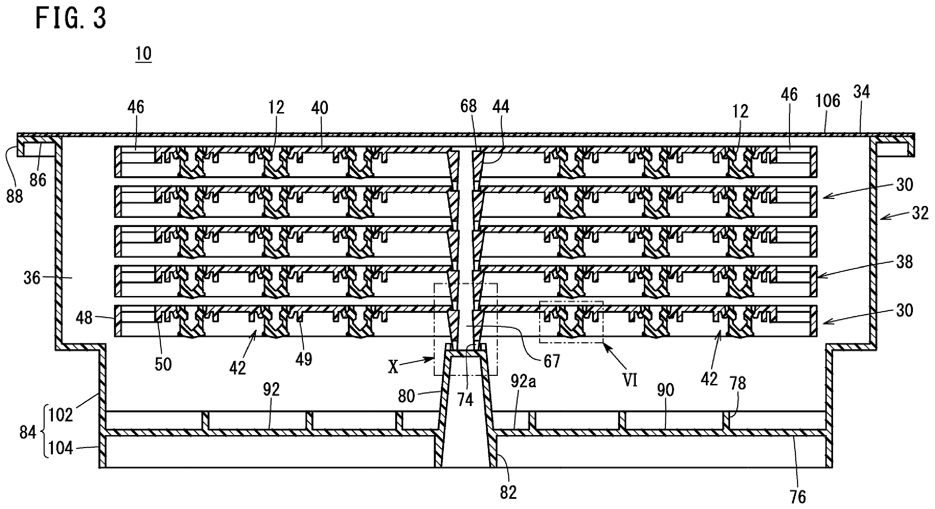

As illustrated in Figs. 2 and 3, the plurality of gasket holding tool sets 30 are housed in the housing chamber 36 of the container 32 in a state of being stacked on each other in the vertical direction. The gasket holding tool set 30 includes a large number of gaskets 12 and a gasket holding tool 38. The gasket holding tool 38 holds a large number of gaskets 12 of the syringe 300. Before being held by the gasket holding tool 38, the outer surface of the gasket 12 including the distal-end-side protruding portion 16 is coated with a lubricant.

The gasket holding tool 38 is integrally molded with a resin material. The gasket holding tool 38 includes a base plate 40, a large number of holding structures 42, and a plurality of leg portions 44. In Fig. 2, the base plate 40 is formed in a quadrangular shape. The base plate 40 has a pair of first sides 45a extending in parallel with each other and a pair of second sides 45b extending in parallel with each other.

Two through-holes 46 for transportation are formed in the base plate 40. The through-hole 46 is a quadrangular hole. The two through-holes 46 are provided so as to be adjacent to the pair of second sides 45b of the base plate 40. The through-hole 46 is formed in a size that allows a finger of a human, a nail of a robot hand, or the like to pass through. The position, size, and shape of the through-hole 46 may be designed appropriately.

As illustrated in Figs. 2 and 3, a reinforcing outer peripheral rib 48 protruding downward (in the thickness direction of the base plate 40) is provided on the outer peripheral portion of the base plate 40. The outer peripheral rib 48 annularly extends along the outer peripheral portion of the base plate 40. However, the outer peripheral rib 48 may not be integrally connected in an annular shape. The outer peripheral rib 48 may be intermittently provided along the outer peripheral portion of the base plate 40.

The outer peripheral rib 48 extends so as to cover the through-hole 46 from the outside. That is, the through-hole 46 is located adjacent to the inner side of the outer peripheral rib 48. The through-hole 46 is surrounded by the outer peripheral rib 48 and a U-shaped reinforcing rib 50. The reinforcing rib 50 protrudes downward from the base plate 40 and is connected to the outer peripheral rib 48.

As illustrated in Figs. 3 and 6, on the base plate 40, an annular protrusion 49 protrudes downward so as to surround the holding structure 42. As a result, the rigidity of the portion of the base plate 40 where the holding structure 42 is provided can be improved by the annular protrusion 49. The protruding length of the annular protrusion 49 is shorter than the entire length of the gasket body 14 in the axial direction.

In Fig. 2, the large number of holding structures 42 are arranged at intervals (at equal intervals) on the base plate 40. The large number of holding structures 42 are arranged in a line along the extending direction of the first side 45a of the base plate 40. The large number of holding structures 42 are arranged in a staggered manner (staggered) along the extending direction of the second side 45b. The holding structure 42 holds the gasket 12.

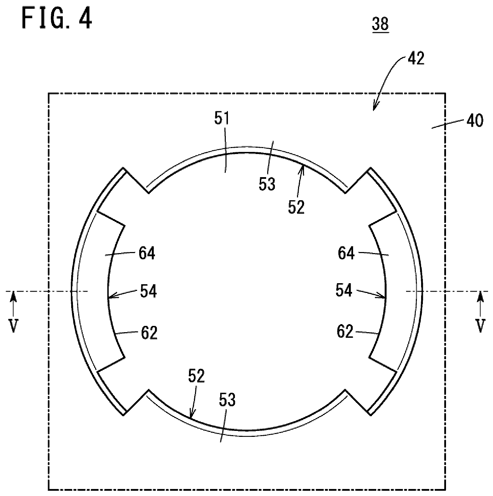

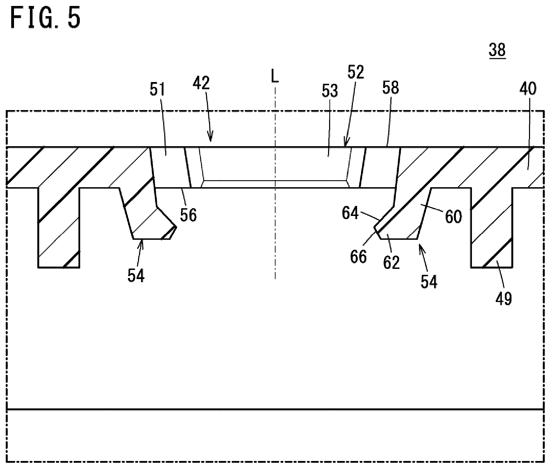

As illustrated in Figs. 4 and 5, the holding structure 42 includes an insertion hole 51, two first holding portions 52, and two second holding portions 54. The insertion hole 51 penetrates the base plate 40 in the thickness direction. The insertion hole 51 has a size and a shape into which the proximal end portion of the gasket 12 can be inserted. The diameter (hole diameter) of the insertion hole 51 is larger than the maximum outer diameter of the gasket 12 (the outer diameter of the distal-end-side protruding portion 16). The insertion hole 51 includes a first opening 56 that opens downward and a second opening 58 that opens upward.

As illustrated in Fig. 4, the two first holding portions 52 are provided at intervals along the circumferential direction of the insertion hole 51. The two first holding portions 52 are disposed so as to face each other. In other words, the two first holding portions 52 are arranged to be shifted by 180° in the circumferential direction of the insertion hole 51. The first holding portion 52 extends in an arc shape along the circumferential direction of the insertion hole 51.

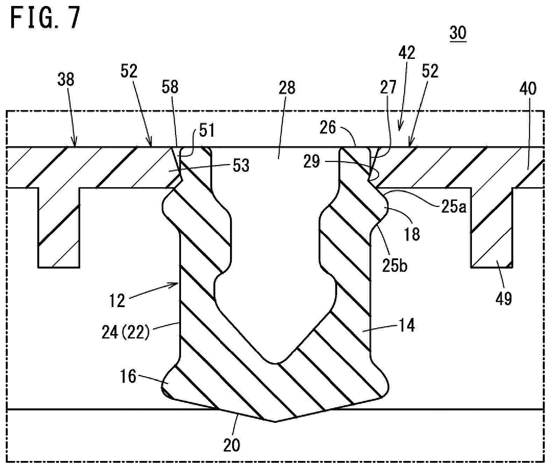

In Figs. 5 and 7, the first holding portion 52 has a first contact portion 53 protruding radially inward from an inner surface forming the insertion hole 51. The first contact portion 53 is formed to be thin in the protruding direction of the first contact portion 53. The protruding end surface of the first contact portion 53 is inclined downward in the protruding direction of the first contact portion 53.

As illustrated in Fig. 4, the two second holding portions 54 are provided at intervals along the circumferential direction of the insertion hole 51. The two second holding portions 54 are disposed so as to face each other. In other words, the two second holding portions 54 are arranged to be shifted by 180° in the circumferential direction of the insertion hole 51. The second holding portion 54 extends in an arc shape along the circumferential direction of the insertion hole 51. The length of the second holding portion 54 along the circumferential direction of the insertion hole 51 is shorter than the length of the first holding portion 52 along the circumferential direction of the insertion hole 51. However, the lengths of the first holding portion 52 and the second holding portion 54 along the circumferential direction of the insertion hole 51 can be appropriately set.

In Fig. 5, the second holding portion 54 includes an extending portion 60 extending downward from the base plate 40 and a second contact portion 62 protruding from the extending end portion of the extending portion 60 toward a center line L of the insertion hole 51. The base portion of the extending portion 60 is located in a portion of the lower surface of the base plate 40 adjacent to the insertion hole 51. The extending portion 60 is inclined downward so as to approach the center line L.

The second contact portion 62 is located below the first opening 56. In other words, the upper end of the second contact portion 62 is located below the lower end of the first holding portion 52. The second contact portion 62 is formed to be thin in the protruding direction of the second contact portion 62. The protruding end surface of the second contact portion 62 includes a contact surface 64 facing upward and a tapered surface 66 facing downward. The contact surface 64 is inclined downward in the protruding direction of the second contact portion 62. The tapered surface 66 is inclined upward in the protruding direction of the second contact portion 62. In other words, the tapered surface 66 is inclined downward in a direction (outward) away from the center line L of the insertion hole 51.

As illustrated in Fig. 4, when viewed from the thickness direction of the base plate 40 (in top view), the two first holding portions 52 and the two second holding portions 54 are alternately arranged so as not to overlap each other. In Fig. 5, the first contact portion 53 and the second contact portion 62 are located so as to be offset from each other in the thickness direction of the base plate 40.

In a case where the gasket 12 is attached to the holding structure 42, as illustrated in Fig. 6, the gasket 12 is inserted into the insertion hole 51 from the first opening 56 in a state where the proximal end surface 26 of the gasket body 14 faces the first opening 56 of the insertion hole 51. Then, a surface 25a of the proximal-end-side protruding portion 18 facing the proximal direction comes into contact with the tapered surface 66 of the second holding portion 54 and presses the tapered surface 66 toward the insertion hole 51. As a result, the two second holding portions 54 are bent in directions away from each other, so that the proximal-end-side protruding portion 18 gets over the second contact portion 62 of the second holding portion 54.

Then, the second contact portion 62 of the second holding portion 54 abuts on a surface 25b of the proximal-end-side protruding portion 18 facing the distal direction in a state where the gasket 12 is inserted into the insertion hole 51. As illustrated in Fig. 7, the first contact portion 53 of the first holding portion 52 abuts on a proximal-end-side outer peripheral surface 27 of the outer peripheral surface 22 of the gasket body 14 located in the proximal direction with respect to the proximal-end-side protruding portion 18 in a state where the gasket 12 is inserted into the insertion hole 51. In other words, the first holding portion 52 presses the proximal-end-side outer peripheral surface 27 of the gasket body 14 radially inward in a state where the gasket 12 is inserted into the insertion hole 51. In the present embodiment, in a state where the gasket 12 is inserted into the insertion hole 51, the first holding portion 52 abuts on an adjacent portion 29 adjacent in the proximal direction to the proximal-end-side protruding portion 18 of the proximal-end-side outer peripheral surface 27 of the gasket body 14.

Accordingly, the gasket 12 is held in a predetermined posture by the first holding portion 52 and the second holding portion 54. In a case where the gasket 12 is attached to the holding structure 42 in this manner, the distal-end-side protruding portion 16 of the gasket 12 does not touch the holding structure 42. Therefore, the distal-end-side protruding portion 16 is prevented from coming into contact with the holding structure 42 and being damaged (for example, peeling off the coating of the lubricant).

In the holding state (hereinafter, it may be simply referred to as a "holding state of the gasket 12") in which the gasket 12 is held by the holding structure 42, the distal-end-side protruding portion 16 is away from (not in contact with) the holding structure 42 (the first holding portion 52 and the second holding portion 54). In the holding state of the gasket 12, the distal end (lower end) of the gasket 12 protrudes downward from the lower end of the annular protrusion 49.

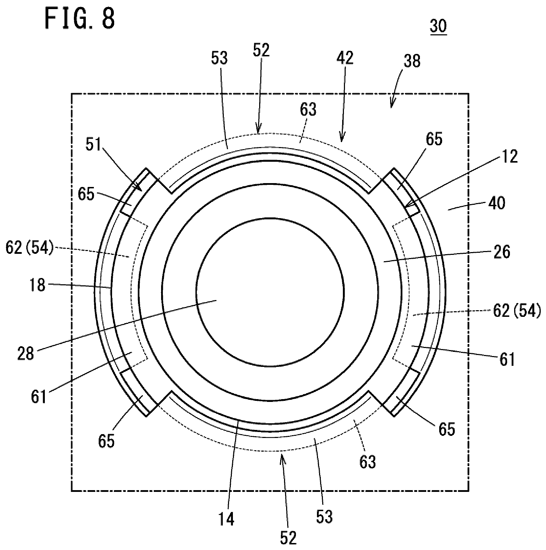

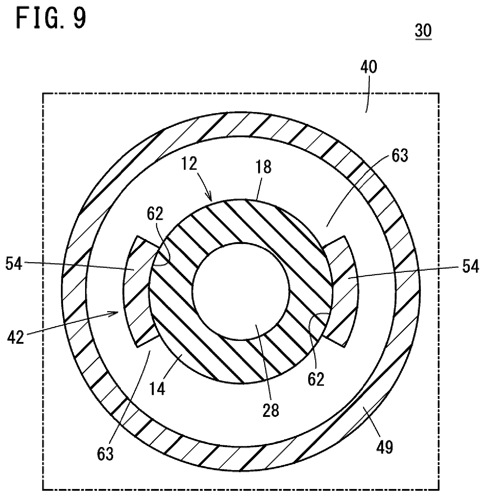

As illustrated in Fig. 8, in the holding state of the gasket 12, a first gap 61 through which steam can flow is formed between the first holding portions 52 adjacent to each other. As illustrated in Fig. 9, in the holding state of the gasket 12, a second gap 63 through which steam can flow is formed between the second holding portions 54 adjacent to each other. As understood from Fig. 8, in the holding state of the gasket 12, when viewed from the thickness direction of the base plate 40 (in top view), a communication passage 65 formed by vertically connecting the first gap 61 and the second gap 63 is formed between the first holding portion 52 and the second holding portion 54. Steam can smoothly flow through the communication passage 65 in the vertical direction. The communication passages 65 are located at four positions in the circumferential direction of the gasket 12.

The number, position, shape, and size of each of the first holding portion 52 and the second holding portion 54 can be appropriately set. In particular, the numbers of the first holding portions 52 and the second holding portions 54 may be one or more (other than two). In a case where the number of first holding portions 52 is one, the first holding portion 52 may extend in a range of 180° or more and less than 360° in the circumferential direction of the insertion hole 51 so as to form a gap for steam flow (for example, it may extend in a C shape or a U shape). The same applies to the second holding portion 54. As the numbers of the first holding portions 52 and the second holding portions 54 increase, the gasket 12 can be stably held, but the gap for the steam flow narrows, and it becomes difficult to remove the gasket 12 from the gasket holding tool 38.

In Fig. 2, the leg portions 44 are provided at a central portion and an outer peripheral portion of the base plate 40. In the example of Fig. 2, one leg portion 44 is provided in the central portion of the base plate 40 and six leg portions are provided in the outer peripheral portion of the base plate 40. The number and positions of the leg portions 44 can be set as appropriate.

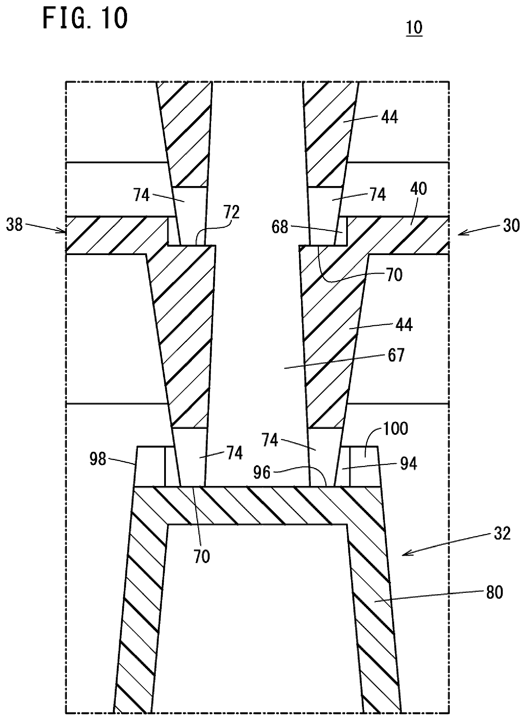

As illustrated in Fig. 10, the leg portion 44 protrudes downward from the base plate 40. The leg portion 44 is formed in a cylindrical shape. That is, the leg portion 44 has an inner hole 67 penetrating in the vertical direction. The outer peripheral surface of the leg portion 44 is tapered in diameter toward the lower side (protruding direction). As illustrated in Fig. 3, the lower end of the leg portion 44 is located below the lower end of the annular protrusion 49. The lower end of the leg portion 44 is located below the lower end (distal end) of the gasket 12 in the holding state of the gasket 12.

In Fig. 10, a leg insertion recess 68 into which the lower end of the leg portion 44 is insertable is provided at the upper end of the leg portion 44. The leg insertion recess 68 is a circular recess. The inner hole 67 of the leg portion 44 is opened in a bottom surface 72 of the leg insertion recess 68. That is, the bottom surface 72 of the leg insertion recess 68 extends in an annular shape. With the gasket holding tool 38 stacked in the vertical direction, a lower end surface 70 of the leg portion 44 abuts on the bottom surface 72 of the leg insertion recess 68. The plurality of gasket holding tools 38 are located in the horizontal direction by inserting the leg portion 44 into the leg insertion recess 68.

Two notches 74 for discharging water (liquid water) in the inner hole 67 of the leg portion 44 are formed at the lower end of the leg portion 44. The two notches 74 are located at positions shifted by 180° in the circumferential direction of the leg portion 44. The number, position, size, and shape of the notches 74 may be designed appropriately.

As illustrated in Figs. 2 and 3, the container 32 is integrally molded with a resin material. The container 32 has a housing chamber 36 that houses the plurality of gasket holding tool sets 30.

The container 32 includes a bottom wall 76, a bottom surface rib 78, a support portion 80, a container leg portion 82 (see Fig. 3), a peripheral wall 84, a flange portion 86, and a flange rib 88. In Fig. 3, the bottom wall 76 is formed in a plate shape and is connected to an intermediate portion in the height direction of the peripheral wall 84. The bottom wall 76 is located between the center of the peripheral wall 84 in the vertical direction (height direction) and the lower end of the peripheral wall 84. The bottom wall 76 has a bottom surface 90 forming the housing chamber 36.

The bottom surface rib 78 protrudes upward from the bottom surface 90 of the bottom wall 76. In Figs. 2, 3, and 11, the bottom surface rib 78 is provided so as to partition the bottom surface 90 into a plurality of regions 92. Each region 92 can store water generated during high-pressure steam sterilization.

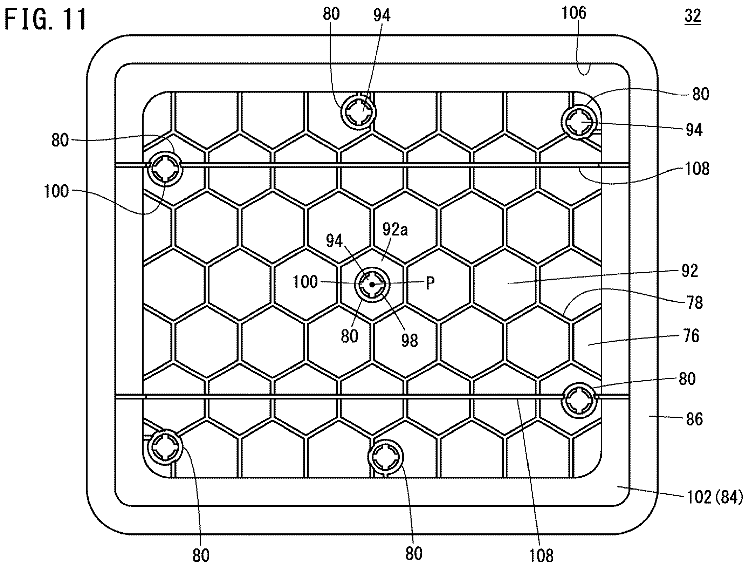

As illustrated in Figs. 2 and 11, the bottom surface rib 78 is formed such that each region 92 has a polygonal shape in top view. Specifically, the bottom surface rib 78 is formed such that each region 92 has a hexagonal shape in top view. In other words, the bottom surface rib 78 has a honeycomb structure in top view.

The size and shape of each region 92, and the number of regions 92 formed by the bottom surface rib 78 can be appropriately set. The bottom surface rib 78 may be formed such that each region 92 has a triangular shape, a quadrangular shape, or a pentagonal shape.

As illustrated in Fig. 11, the bottom surface rib 78 is formed such that one region 92 (central region 92a) of the plurality of regions 92 is located at the center of the bottom wall 76. A center P of the bottom wall 76 is located in the central region 92a. Specifically, the center P of the bottom wall 76 is located at the central portion of the hexagonal region 92. In other words, the center P of the bottom wall 76 is located at the center of the central region 92a or in the vicinity of the center.

As illustrated in Figs. 2 and 3, the support portion 80 protrudes upward from the bottom wall 76 to support portion the leg portion 44 of the gasket holding tool 38. As many support portions 80 as the leg portions 44 of the gasket holding tool 38 are provided (see Fig. 2).

In Figs. 2, 10, and 11, a support recess 94 into which the lower end of the leg portion 44 is insertable is provided at the upper end of the support portion 80. The lower end surface 70 of the leg portion 44 abuts on a bottom surface 96 of the support recess 94 (see Fig. 10). In a side wall 98 forming the support recess 94, four drain holes 100 (drain grooves) for guiding water (liquid water) discharged from the notch 74 to the outside of the support portion 80 are formed. The four drain holes 100 are provided at equal intervals (90° intervals) in the circumferential direction of the support portion 80.

As illustrated in Fig. 3, the container leg portion 82 extends downward from the lower end of the support portion 80. The container leg portions 82 are provided in the same number as the support portions 80. The lower surface of the container leg portion 82 is at the same height position as the lower end of the peripheral wall 84. The lower surface of the container leg portion 82 comes into contact with an arrangement surface of a chamber (surface on which the gasket housing body 10 is arranged) of a high-pressure steam sterilization device (not illustrated). The support portion 80 and the container leg portion 82 receive the weight of the plurality of gasket holding tool sets 30.

In Figs. 2 and 3, the peripheral wall 84 includes an upper peripheral wall 102 extending upward from the outer peripheral portion of the bottom wall 76 and a lower peripheral wall 104 extending downward from the outer peripheral portion of the bottom wall 76. The upper peripheral wall 102 extends in a quadrangular shape so as to surround the plurality of gasket holding tool sets 30 from the horizontal direction. The upper peripheral wall 102 forms the housing chamber 36. The upper peripheral wall 102 is bent so as to expand outward at a middle portion extending upward from the bottom wall 76. Accordingly, the rigidity of the upper peripheral wall 102 can be enhanced. An opening 106 is formed at an upper end of the upper peripheral wall 102. A plate-shaped rib 108 is provided on the inner surface of the peripheral wall 84 and the bottom surface 90 of the bottom wall 76 (see Fig. 2).

The flange portion 86 extends outward from the upper end of the upper peripheral wall 102. The flange portion 86 extends annularly and is formed in a plate shape. The sealing member 34 is fixed to the upper surface of the flange portion 86. The flange rib 88 protrudes downward from the extending end of the flange portion 86. The flange rib 88 reinforces the flange portion 86. The flange rib 88 extends annularly so as to surround the flange portion 86. The flange rib 88 only needs to protrude in at least one of the vertical directions from the flange portion 86.

The sealing member 34 seals the opening 106 of the container 32. The sealing member 34 is formed to allow vapor to pass therethrough.

In the present embodiment, as illustrated in Fig. 3, the plurality of gasket holding tool sets 30 are arranged on the support portion 80 in a state of being overlapped with each other. At this time, the lower end of the leg portion 44 of the gasket holding tool 38 located at the lowermost position is inserted into the support recess 94 of the support portion 80 (see Fig. 10). Therefore, the gasket holding tool 38 can be easily located with respect to the container 32.

Further, the lower end portion of the leg portion 44 of the gasket holding tool 38 placed on the lowermost gasket holding tool 38 is inserted into the leg insertion recess 68 of the gasket holding tool 38 adjacent below (see Fig. 10). Thus, the plurality of gasket holding tool sets 30 can be easily located. After the plurality of gasket holding tool sets 30 are arranged in the housing chamber 36 of the container 32, the opening 106 of the container 32 is sealed by the sealing member 34.

The gasket housing body 10 is subjected to high-pressure steam sterilization in a chamber of a high-pressure steam sterilization device (not illustrated). The steam in the chamber passes through the sealing member 34, flows into the housing chamber 36 of the container 32, and comes into contact with the gasket 12. At this time, since the steam flows through the first gap 61, the second gap 63, and the communication passage 65 (see Figs. 8 and 9), the steam smoothly flows around the gasket 12. The water (liquid water) generated in the housing chamber 36 is dispersed and stored in the plurality of regions 92 of the bottom surface 90. That is, the water is not unevenly stored in one place of the bottom surface 90. Part of the water stored in each region 92 evaporates and returns to steam.

Water (liquid water) generated in the inner hole 67 of the leg portion 44 is guided to the inner hole 67 of the leg portion 44 of the gasket holding tool 38 located at the lowermost position. The water in the inner hole 67 of the leg portion 44 is discharged to the outside of the support portion 80 via the notch 74 and the drain hole 100 and stored in the region 92 of the bottom surface 90.

After the high-pressure steam sterilization is performed, the sealing member 34 is opened. Then, a human finger or a claw of a robot arm is inserted into the through-hole 46 of the gasket holding tool 38, and the gasket holding tool set 30 is transported to a plugging device (not illustrated).

Thereafter, for example, the outer tube 310 (barrel) of the syringe 300 is disposed below the gasket 12 of the gasket holding tool set 30 in a state where the proximal end opening of the outer tube 310 faces upward. Then, the gasket 12 is plugged (for example, a vacuum plug) into the outer tube 310 by pressing the proximal end surface 26 of the gasket 12 from above toward the proximal end opening of the outer tube 310. As a result, the syringe 300 in which the gasket 12 is inserted into the outer tube 310 is manufactured.

The present embodiment has the following effects.

According to the present embodiment, by inserting the gasket 12 into the insertion hole 51 from the proximal direction of the gasket body 14, the gasket 12 can be held by the first holding portion 52 and the second holding portion 54 without the distal-end-side protruding portion 16 which is the sealing portion coming into contact with the holding structure 42. In addition, the holding structure 42 is not in contact with the distal-end-side protruding portion 16 (sealing portion) in the holding state of the gasket 12. As a result, the gasket 12 can be held by the gasket holding tool 38 while suppressing the distal-end-side protruding portion 16 from being damaged by being in contact with the holding structure 42. Further, since the first contact portion 53 abuts on the proximal-end-side outer peripheral surface 27 of the gasket body 14 and the second contact portion 62 abuts on the surface 25a of the proximal-end-side protruding portion 18 of the gasket 12 facing the proximal direction, the gasket 12 can be stably held.

A plurality of first holding portions 52 and a plurality of second holding portions 54 are provided at intervals along the circumferential direction of the insertion hole 51. The first gap 61 is formed between the first holding portions 52 adjacent to each other in the holding state of the gasket 12. The second gap 63 is formed between the second holding portions 54 adjacent to each other in the holding state of the gasket 12. Steam can flow through the first gap 61 and the second gap 63 when the gasket 12 is sterilized with high-pressure steam.

According to such a configuration, the gasket 12 held by the gasket holding tool 38 can be efficiently sterilized with high-pressure steam. In addition, the gasket 12 can be more stably held by the plurality of first holding portions 52 and the plurality of second holding portions 54.

The plurality of first holding portions 52 and the plurality of second holding portions 54 are alternately arranged so as not to overlap each other in the circumferential direction of the insertion hole 51 when viewed from the thickness direction of the base plate 40.

According to such a configuration, the steam can be smoothly circulated around the gasket 12.

The first holding portion 52 is provided on the inner surface of the insertion hole 51. The second holding portion 54 includes an extending portion 60 extending from the base plate 40 in the thickness direction of the base plate 40, and a second contact portion 62 protruding from the extending end portion of the extending portion 60 toward the center line L of the insertion hole 51.

According to such a configuration, the holding structure 42 can be formed compactly.

The second contact portion 62 has a tapered surface 66 inclined in a direction away from the center line L of the insertion hole 51 toward a direction opposite to the first contact portion 53.

According to such a configuration, by bringing the proximal-end-side protruding portion 18 of the gasket 12 into contact with the tapered surface 66, the proximal-end-side protruding portion 18 easily gets over the second contact portion 62.

The gasket holding tool 38 includes a leg portion 44 protruding downward from the base plate 40. The lower end of the leg portion 44 is located below the lower end of the gasket 12 in the holding state.

According to such a configuration, in a case where the plurality of gasket holding tools 38 are disposed in an overlapping manner, it is possible to prevent the adjacent gasket holding tools 38 from coming into contact with the gasket 12.

A leg insertion recess 68 into which a lower end of the leg portion 44 is insertable is provided at an upper end of the leg portion 44.

According to such a configuration, in a case where the plurality of gasket holding tools 38 are disposed in an overlapping manner, the lower end portion of the leg portion 44 can be easily located by being inserted into the leg insertion recess 68.

The leg portion 44 is formed in a tubular shape, and a notch 74 for discharging water inside the leg portion 44 is formed at a lower end portion of the leg portion 44.

According to such a configuration, in a case where the gasket 12 is sterilized with high-pressure steam, the water (liquid water) in the leg portion 44 can be discharged to the outside from the notch 74.

An outer peripheral rib 48 protruding in a thickness direction of the base plate 40 is provided on an outer peripheral portion of the base plate 40.

According to such a configuration, the rigidity of the outer peripheral portion of the base plate 40 can be improved by the outer peripheral rib 48.

A through-hole 46 for transportation is formed in a portion of the base plate 40 adjacent to the outer peripheral rib 48.

According to such a configuration, it is possible to easily transport the gasket holding tool 38 by inserting a human finger, a claw of a robot arm, or the like into the through-hole 46. Further, the periphery of the portion of the base plate 40 where the through-hole 46 is formed can be reinforced by the outer peripheral rib 48.

In the gasket housing body 10, the container 32 includes a bottom wall 76 having a bottom surface 90 forming the housing chamber 36 of the container 32, a peripheral wall 84 provided on an outer peripheral portion of the bottom wall 76, and a bottom surface rib 78 protruding upward from the bottom surface 90. The bottom surface rib 78 is provided so as to partition the bottom surface 90 into a plurality of regions 92.

According to such a configuration, the rigidity of the bottom wall 76 can be improved by the bottom surface rib 78. Therefore, thermal deformation of the bottom wall 76 (warpage of the bottom wall 76) during high-pressure steam sterilization can be suppressed. In addition, water (liquid water) generated at the time of high-pressure steam sterilization of the gasket 12 can be dispersed and stored in the plurality of regions 92. That is, it is possible to suppress biased accumulation of water (liquid water) in the bottom surface 90.

The bottom surface rib 78 is formed such that each of the plurality of regions 92 has a polygonal shape in top view.

According to such a configuration, the rigidity of the bottom wall 76 can be effectively improved by the bottom surface rib 78.

The bottom surface rib 78 is formed such that each of the plurality of regions 92 has a hexagonal shape in top view.

According to such a configuration, the rigidity of the bottom wall 76 can be more effectively improved by the bottom surface rib 78. In addition, the plurality of regions 92 can be arranged on the bottom surface 90 in a well-balanced manner.

The bottom surface rib 78 is formed such that one region 92 (central region 92a) of the plurality of regions 92 is located at the center of the bottom wall 76. The center P of the bottom wall 76 is located in the central region 92a.

According to such a configuration, since the bottom surface rib 78 can be arranged on the bottom surface 90 in a more balanced manner, it is possible to suppress the variation in the magnitude of the rigidity of the bottom wall 76. This makes it possible to further suppress thermal deformation of the bottom wall 76 (warpage of the bottom wall 76) at the time of high-pressure steam sterilization.

The bottom wall 76 is located at a vertically intermediate portion of the peripheral wall 84.

According to such a configuration, the rigidity of the peripheral wall 84 can be effectively improved by the bottom wall 76 as compared with the case where the bottom wall 76 is located at the lower end of the peripheral wall 84.

The container 32 has a flange portion 86 extending outward from an upper end portion of the peripheral wall 84 and a flange rib 88 protruding in the vertical direction from the flange portion 86.

According to such a configuration, the rigidity of the upper end portion of the peripheral wall 84 can be improved by the flange portion 86 and the flange rib 88.

The container 32 has a support portion 80 protruding upward from the bottom wall 76. A support recess 94 into which the lower end of the leg portion 44 is insertable is provided at the upper end of the support portion 80.

According to such a configuration, the weight of the gasket holding tool set 30 can be supported by the support portion 80 protruding from the bottom wall 76. By inserting the lower end portion of the leg portion 44 of the gasket holding tool 38 into the support recess 94, the gasket holding tool 38 can be easily located with respect to the container 32.

The lower end surface 70 of the leg portion 44 abuts on the bottom surface 96 of the support recess 94. In the side wall 98 forming the support recess 94, a drain hole 100 for guiding the water discharged from the notch 74 to the outside of the support portion 80 is formed.

According to such a configuration, the water (liquid water) in the leg portion 44 can be discharged through the notch 74 and the drain hole 100 at the time of high-pressure steam sterilization of the gasket 12.

The container 32 has a container leg portion 82 that extends downward from the support portion 80 below the bottom wall 76 and receives a load acting on the support portion 80.

According to such a configuration, since the load acting on the support portion 80 can be received by the container leg portion 82, it is possible to suppress the support portion 80 and the bottom wall 76 from being deformed by the weight of the gasket holding tool set 30.

### (First modification)

Next, a holding structure 42a according to a first modification will be described. Further, in the present modification, the same components as those of the above-described holding structure 42 are denoted by the same reference numerals, and the detailed description thereof will be omitted.

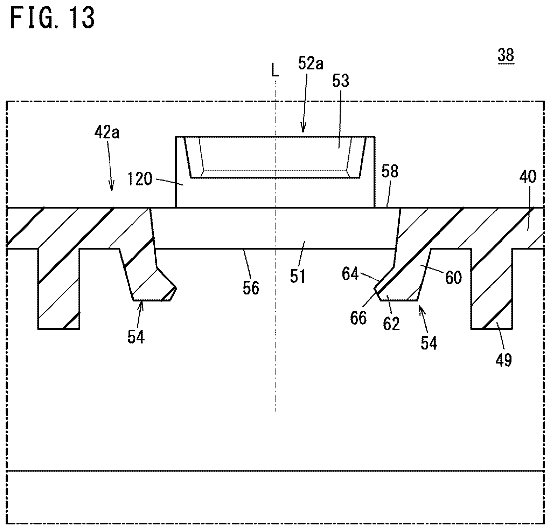

As illustrated in Fig. 13, the holding structure 42a includes two first holding portions 52a and two second holding portions 54. The first holding portion 52a includes an extending portion 120 extending upward from the base plate 40 and a first contact portion 53 protruding from an extending end portion of the extending portion 120 toward the center line L of the insertion hole 51. The holding structure 42a according to the present modification has the same effect as the holding structure 42 described above.

### (Second modification)

Next, a holding structure 42b according to a second modification will be described. Further, in the present modification, the same components as those of the above-described holding structures 42 and 42a are denoted by the same reference numerals, and the detailed description thereof will be omitted.

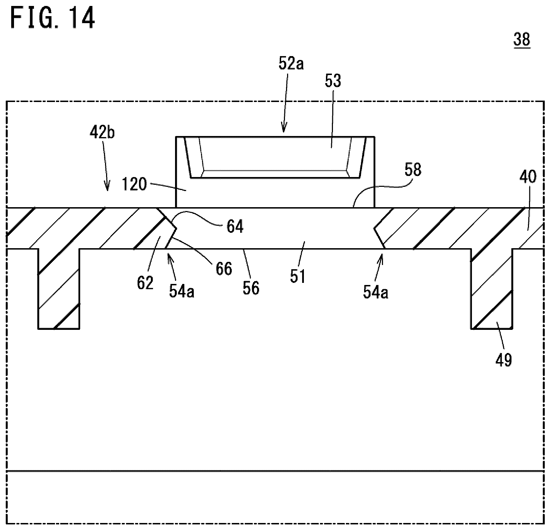

As illustrated in Fig. 14, the holding structure 42b includes two first holding portions 52a and two second holding portions 54a. The second holding portion 54a is provided on the inner peripheral surface of the insertion hole 51. The second holding portion 54a has the second contact portion 62 described above. That is, the second holding portion 54a has the contact surface 64 and the tapered surface 66. The holding structure 42b according to the present modification has the same effect as the holding structure 42 described above.

In the present embodiment, an example has been described in which the gasket holding tool 38 is used in both the step of sterilizing the gasket 12 with high-pressure steam (high-pressure steam sterilization step) and the step of inserting the gasket 12 into the outer tube 310 of the syringe 300 (plugging step). However, the gasket holding tool 38 may not be used in the high-pressure steam sterilization step, and may be used only in the plugging step.

In the present embodiment, the high-pressure steam sterilization has been described as an example of the sterilization treatment, but gas sterilization may be performed instead of the high-pressure steam sterilization. Examples of the gas used for gas sterilization include ethylene oxide and hydrogen peroxide.

Note that the present invention is not limited to the above disclosure, and various configurations can be adopted without departing from the gist of the present invention.

### Reference Signs List

10 gasket housing body
12 gasket
14 gasket body
16 distal-end-side protruding portion (sealing portion)
18 proximal-end-side protruding portion (protruding portion)
22 outer peripheral surface
27 proximal-end-side outer peripheral surface
30 gasket holding tool set
32 container
34 sealing member
36 housing chamber
38 gasket holding tool
40 base plate
42, 42a, 42b holding structure
44 leg portion
46 through-hole
48 outer peripheral rib
51 insertion hole
52, 52a first holding portion
53 first contact portion
54, 54a second holding portion
60 extending portion
61 first gap
62 second contact portion
63 second gap
66 tapered surface
68 leg insertion recess
70 lower end surface
74 notch
76 bottom wall
78 bottom surface rib
80 support portion
82 container leg portion
84 peripheral wall
86 flange portion
88 flange rib
92 region
92a central region
94 support recess
98 side wall
100 drain hole
106 opening
300 syringe
310 outer tube
L center line
P center

## Claims

1. A gasket holding tool for holding a gasket to be inserted into an outer tube of a syringe,
the gasket comprising:
a gasket body;
an annular sealing portion provided on an outer peripheral surface of the gasket body and in liquid-tight or gas-tight contact with an inner peripheral surface of the outer tube; and
an annular protruding portion protruding radially outward from a portion of the outer peripheral surface of the gasket body in a proximal direction with respect to the sealing portion,
the gasket holding tool comprising:
a base plate; and
a holding structure that is provided on the base plate and holds the gasket,
wherein
the holding structure comprises:
an insertion hole through which the gasket is inserted;
a first holding portion comprising a first contact portion that abuts on a proximal-end-side outer peripheral surface in the proximal direction with respect to the protruding portion of the outer peripheral surface of the gasket body in a state where the gasket is inserted into the insertion hole; and
a second holding portion comprising a second contact portion that abuts on a surface of the protruding portion facing a distal direction in a state where the gasket is inserted into the insertion hole,
the first contact portion and the second contact portion are located so as to be offset from each other in a thickness direction of the base plate, and
the holding structure is not in contact with the sealing portion in a holding state of holding the gasket.

2. The gasket holding tool according to claim 1, wherein
a plurality of the first holding portions and a plurality of the second holding portions are provided at intervals along a circumferential direction of the insertion hole,
a first gap is formed between the first holding portions adjacent to each other in the holding state,
a second gap is formed between the second holding portions adjacent to each other in the holding state, and
steam or gas can flow through the first gap and the second gap when the gasket is subjected to high-pressure steam sterilization or gas sterilization.

3. The gasket holding tool according to claim 2, wherein a plurality of the first holding portions and a plurality of the second holding portions are alternately arranged so as not to overlap each other when viewed from the thickness direction of the base plate.

4. The gasket holding tool according to claim 1, wherein
one of the first holding portion and the second holding portion is provided on an inner surface of the insertion hole, and
the other one of the first holding portion and the second holding portion extends from the base plate in the thickness direction of the base plate.

5. The gasket holding tool according to claim 1, wherein the second contact portion has a tapered surface inclined in a direction away from a center line of the insertion hole toward a direction opposite to the first contact portion.

6. The gasket holding tool according to claim 1, comprising a leg portion protruding downward from the base plate,
wherein a lower end of the leg portion is located below a lower end of the gasket in the holding state.

7. The gasket holding tool according to claim 6, wherein a leg insertion recess into which a lower end of the leg portion is insertable is provided at an upper end of the leg portion.

8. The gasket holding tool according to claim 6, wherein
the leg portion is formed in a tubular shape, and
a notch for discharging water inside the leg portion is formed at a lower end of the leg portion.

9. The gasket holding tool according to claim 1, wherein an outer peripheral rib protruding in the thickness direction of the base plate is provided on an outer peripheral portion of the base plate.

10. The gasket holding tool according to claim 9, wherein a through-hole for transportation is formed in a portion of the base plate adjacent to the outer peripheral rib.

11. A gasket holding tool set comprising:
a gasket holding tool according to any one of claims 1 to 10; and
the gasket.

12. A gasket housing body comprising:
a gasket holding tool set according to claim 11;
a container that has an opening opened upward and houses the gasket holding tool set; and
a sealing member that seals the opening of the container,
wherein the sealing member is formed such that steam or gas for performing high-pressure steam sterilization or gas sterilization of the gasket can pass therethrough.

13. The gasket housing body according to claim 12, wherein
the container comprises:
a bottom wall having a bottom surface forming a housing chamber of the container;
a peripheral wall provided on an outer peripheral portion of the bottom wall; and
a bottom surface rib protruding upward from the bottom surface, and
the bottom surface rib is provided so as to partition the bottom surface into a plurality of regions.

14. The gasket housing body according to claim 13, wherein the bottom surface rib is formed such that each of the plurality of regions has a polygonal shape in top view.

15. The gasket housing body according to claim 14, wherein the bottom surface rib is formed such that each of the plurality of regions has a hexagonal shape in top view.

16. The gasket housing body according to claim 15, wherein
the bottom surface rib is formed such that one of the plurality of regions is located at a center of the bottom wall, and
a center of the bottom wall is located in the one region.

17. The gasket housing body according to claim 13, wherein the bottom wall is located at an intermediate portion of the peripheral wall in a vertical direction.

18. The gasket housing body according to claim 13, wherein the container comprises:
a flange portion extending outward from an upper end portion of the peripheral wall; and
a flange rib protruding in a vertical direction from the flange portion.

19. A gasket housing body comprising:
a gasket holding tool set comprising a gasket holding tool according to claim 6 and the gasket;
a container that has an opening opened upward and houses the gasket holding tool set; and
a sealing member that seals the opening of the container,
wherein
the sealing member is formed such that steam or gas for performing high-pressure steam sterilization or gas sterilization of the gasket can pass therethrough,
the container comprises:
a bottom wall having a bottom surface forming a housing chamber of the container;
a peripheral wall provided on an outer peripheral portion of the bottom wall; and
a support portion protruding upward from the bottom wall, and
a support recess into which a lower end portion of the leg portion is insertable is provided at an upper end portion of the support portion.

20. The gasket housing body according to claim 19, wherein the leg portion is formed in a tubular shape,
a notch for discharging water inside the leg portion is formed at a lower end portion of the leg portion,
a lower end surface of the leg portion abuts on a bottom surface of the support recess, and
a drain hole for guiding the water discharged from the notch to the outside of the support portion is formed in a side wall forming the support recess.

21. The gasket housing body according to claim 20, wherein the container comprises a container leg portion that extends downward from the support portion below the bottom wall and receives a load acting on the support portion.
